# EUROPEAN PATENT SPECIFICATION

(11) **EP 2 515 104 B1**
(45) Date of publication and mention of the grant of the patent: **23.02.2022**
(21) Application number: 10837085.9
(22) Date of filing: 14.12.2010
(51) Int. Cl.: G01N 27/403, G01N 33/493, G01N 27/416

(54) **PORTABLE DEVICE FOR ANALYSING PH BY ELECTROCHEMICAL MEASUREMENT**
TRAGBARE VORRICHTUNG ZUR ANALYSE DES PH-WERTES MIT ELEKTROCHEMISCHER MESSUNG
DISPOSITIF PORTATIF D'ANALYSE DU pH PAR MESURE ÉLECTROCHIMIQUE

(30) Priority: 14.12.2009 ES 200930750 U
(43) Date of publication of application: 24.10.2012
(73) Proprietor: Universitat de les Illes Balears, 07122 Palma de Mallorca (ES)
(72) Inventor: GOMILA MUÑOZ, Isabel, E-07071 Palma de Mallorca - Illes Balears (ES); GRASES FREIXEDAS, Feliciano, E-07071 Palma de Mallorca - Illes Balears (ES); COSTA-BAUZA, Antonia, E-07071 Palma de Mallorca - Illes Balears (ES); PRIETO ALMIRALL, Rafael M., E-07071 Palma de Mallorca - Illes Balears (ES); MARTÍN BECERRA, Eva, E-07121 Palma de Mallorca (ES); HENRÍQUEZ PELÁEZ, Rubén, E-07121 Palma de Mallorca (ES); ISERN AMENGUAL, Bernat, E-07121 Palma de Mallorca (ES); PERELLÓ BERSTARD, Joan, E-07121 Palma de Mallorca (ES)
(74) Representative: ZBM Patents - Zea, Barlocci & Markvardsen
(86) International application number: PCT/ES2010/070828
(87) International publication number: WO 2011/073484

(56) References cited:
- WO-A1-03/046538
- WO-A1-2005/057210
- WO-A2-03/009776
- FR-A1- 2 602 066
- US-A- 4 447 309
- US-A- 4 912 417
- US-A- 5 218 304
- US-A1- 2003 109 805
- US-A1- 2004 132 204
- US-A1- 2005 194 296
- US-A1- 2008 034 845
- US-A1- 2008 061 792

## Description

This invention is a portable device for analysing pH by electrochemical measurement, which makes it possible to carry out pre-diagnoses quickly and easily without the need for specialist staff.

### BACKGROUND OF THE INVENTION

Renal lithiasis is a pathology caused by the crystalisation of different substances in the urinary tract. More specifically, the crystalisation of uric acid always occurs in urine with a pH lower than 5.5. There are various factors that contribute to the formation of kidney stones, as well as hyperuricosuria.

It must be taken into account that the pKa of uric acid is approximately 5.5. This is the pH at which the ionic and non-ionic species of uric acid are balanced. Therefore, urine with a pH lower than 5.5 presents a higher proportion of undissociated (insoluble) forms that facilitate the formation of uric acid crystals and kidney stones.

On the other hand, the crystalisation of calcium phosphate in the form of hydroxyapatite [Ca₁₀(PO₄)₆(OH)₂], always occurs at a urinary pH higher than 6. There are other factors that contribute to the formation of hydroxyapatite stones, such as the presence or absence of crystalisation inhibitors or supersaturation, as a thermodynamic factor.

Finally, the crystalisation of calcium oxalate is caused by heterogeneous nucleation where, at a very high percentage, uric acid crystals or hydroxyapatite crystals can act as heterogeneous nuclei in the formation of calcium oxalate crystals.

One of the problems to be solved is the lack of portable devices that can easily and effectively measure the risk of kidney stones forming (consequence of urinary pH or other altered factors) in the urine of lithiasic patients or in healthy people who wish to prevent calcium oxalate, uric acid and hydroxyapatite lithiasis.

Although this check could be carried out using pH indicator paper or a similar system, we must point out that the precision of said systems is not enough to distinguish 0.1 pH units and therefore it would be difficult to obtain a diagnostic value regarding the risk of developing lithiasis.

In addition, the majority of pH metres with a digital or analogue indicator are difficult for patients to use, interpret and maintain and involve a high cost for individual use.

WO 03/009776 A2 discloses a urine test device comprising a cup with a lid and a colorimetric immuno test strip. WO 2005/057210 A1 comprises a urine collection cup for installation in a toilet bowl. The cup comprises an electrochemical pH sensor installed in the bottom of the cup. US 2005/0194296 A1 and US 5,218,304 disclose electrochemical pH probes comprising electrodes and the complete electronics in one housing.

### DESCRIPTION OF THE INVENTION

To achieve the foregoing objects, this invention is a portable device for analysing pH by electrochemical measurement in accordance with claim 1, the use of a portable analysis device in accordance with claims 7 and 9 as well as a method for measuring pH by electrochemical measurement in accordance with claim 10.

In the dependent claims, preferred embodiments of this invention are defined.

In a preferred embodiment the portable pH analysis device comprises a pH sensor, a microprocessor and display means for the results of the analysis, and is characterised for having three light indicators, the microprocessor being configured to activate:
- the first of these indicators when the pH is lower than an initial pH threshold,
- the second of these indicators when the pH is higher than a second pH threshold and,
- the third of these indicators when the pH is between the abovementioned first and second thresholds.

Thus, this device will be useful to lithiasic patients, in that they can determine for themselves and with precision the effects of a diet and even food, drinks and dietary supplements, medicines or physical activity on the risk of kidney stone formation. More specifically, for patients who present a clear risk of lithiasis from uric acid or hydroxyapatite, as well as for those who are at risk of oxalocalcium lithiasis. We must point out that preventing the crystalisation of uric acid and hydroxyapatite would also indirectly prevent oxalocalcium lithiasis in a very high percentage of cases.

The device in accordance with the invention is configured in the form of an adaptable lid for a urine specimen collection cup, the pH sensor being arranged to enter into contact with a urine sample inside the cup, once the device is affixed to the cup, thus allowing the device to be used under hygienic conditions and providing an effective reading, where possible placing the electrode in a suitable position with regard to the sample.

Ideally, the first pH threshold is 5.5 and the second threshold is 6.

Advantageously, the pH sensor comprises a pH electrode and a reference electrode for measuring the proton electrochemical potential in the urine.

A further benefit is that the device includes means for measuring the temperature in the pH and reference electrodes.

Finally, on the device of the invention, the first indicator is red, the second is blue and the third is green.

The device can be fitted with additional sensors for measuring a variable by electrochemical measurement such as calcium or redox potential.

In a second aspect of the invention, the use of a device is defined in accordance with the first aspect of the invention for measuring pH by electrochemical measurement in a biological fluid sample. The biological fluid is preferably urine, although it may be another fluid, for example blood, saliva or sputum.

In a third aspect of the invention a method of measuring pH by electrochemical measurement of a sample is defined, which involves measuring pH by electrochemical measurement of the sample and providing an indication of the results of the measurement, depending on it being within at least one range of values.

### BRIEF DESCRIPTION OF THE DRAWINGS

In order to achieve a better understanding of what has been presented, some drawings have been attached in which a practical case of use is represented schematically and only as a non-limitative example.

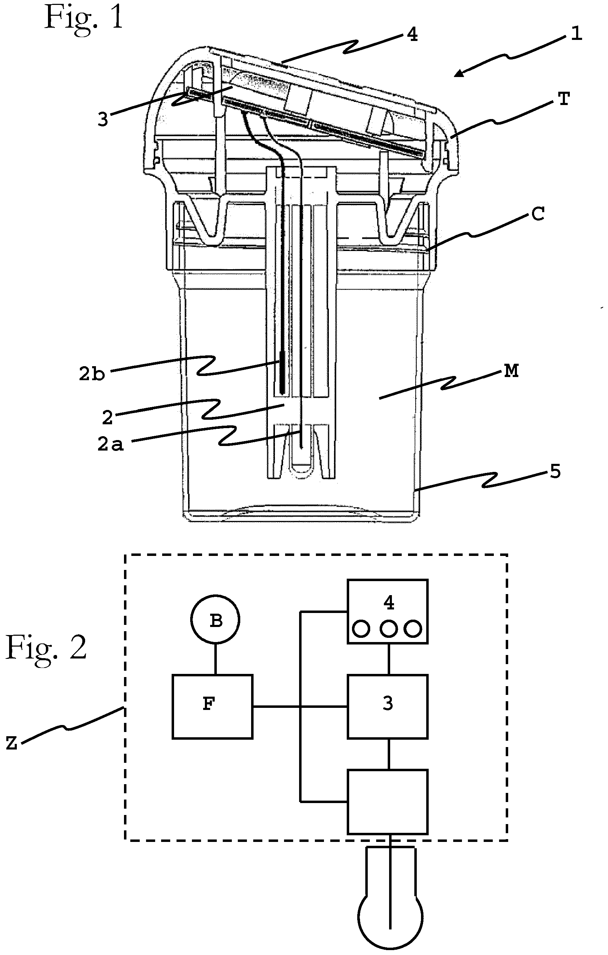

Figure 1 is a cross section of the preferred embodiment of the invention in which it is configured in the form of a lid affixed to a specimen cup.
Figure 2 is a block diagram showing the essential elements of the device of the invention.

### DESCRIPTION OF A PREFERRED EMBODIMENT

As illustrated in the diagrams, in a particular embodiment the invention refers to a portable device 1 for analysing pH in urine M with a pH sensor 2, a microprocessor 3 and display means 4 for the results of the analysis.

More specifically, the invention is characterised for having three light indicators 4a, 4b and 4c, the microprocessor being configured to activate:
- the first of these indicators 4a when the pH is lower than an initial pH threshold,
- the second of these indicators 4b when the pH is higher than a second pH threshold and,
- the third of these indicators 4c when the pH is between the abovementioned first and second thresholds.

Thus, any user can carry out a pre-diagnosis without the need to seek medical advice. Likewise, this could also be a pre-diagnosis tool for medical staff.

In accordance with the preferred embodiment of the invention, the device is configured in the form of a lid T which can be adapted to a urine M specimen cup 5.

The pH sensor 2 is arranged to enter into contact with a urine sample M contained in the cup 5, when the device 1 is attached to the cup 5, in such a way that adequate hygiene conditions are ensured and the correct placement of the electrodes is guaranteed with regards to the urine sample collected in the cup 5.

As has already been specified, the first threshold is 5.5, meaning that a lower reading indicates the capacity of nucleation and formation of uric acid crystals, which at the same time can become growth nuclei for calcium oxalate crystals. The second threshold is 6, meaning that the detection of a pH higher than this second threshold indicates the capacity of nucleation and growth of hydroxyapatite crystals, which can at the same time act as a nucleant for the calcium oxalate crystals.

The pH sensor 2 comprises a pH electrode 2a and a reference electrode 2b for measuring the proton electrochemical potential in the urine.

Calibration of the equipment is very simple, it only being necessary to replace the measuring cup with a test cup, which must contain a standard solution.

Likewise, in accordance with this preferred embodiment, the device contains means for measuring the temperature in the pH electrodes 2a and reference electrodes 2b.

As can be seen in diagram 2, the reading electrodes 2 protrude from the lid, and housed inside the lid is the battery B, the power source F, the electrode impedance adaptor Z, the microprocessor 3 and the indicator lights 4, connected together as shown in the diagram.

Using the device is extremely simple and consists of the following stages:
1. Separate the cup from the rest of the instrument by unscrewing the lid.
2- The patient deposits the urine in the cup
3- Screw the lid back on and shake lightly.
4- Press a test key.
5- In accordance with this preferred embodiment, the three lights will blink in sequence for a few seconds to indicate that the measurement is being taken. The result has been obtained when one of the lights stays lit.
6- Unscrew the lid, throw away the contents and wash both the cup, as well as the lid, with plenty of water before using again.
7- Screw the two parts together again so that some drops of water stay inside.

## Claims

1. Portable device (1) for analysing pH of a sample by electrochemical measurement, which comprises:
• a specimen collection cup (5) configured to contain the sample,
• a lid (T) configured to be affixed to the specimen collection cup (5), and
• a pH electrochemical sensor (2),
wherein the portable device (1) is **characterized in that** the lid (T) comprises:
∘ a microprocessor (3) connected to the pH electrochemical sensor (2) and configured to analyse the pH of the sample based on a measurement from the pH electrochemical sensor (2),
∘ display means (4) connected to the microprocessor (3) to display the results of the analysis,
∘ a power source (F) and/or a battery (B) connected to the microprocessor (3) and the display means (4), and
wherein the pH electrochemical sensor (2) protrudes from the lid (T) to be arranged to enter into contact with the sample contained in the specimen collection cup (5), the pH electrochemical sensor (2) comprising:
∘ a pH electrode (2a), and
∘ a reference electrode (2b).

2. Portable device (1) in accordance with claim 1 in which the display means (4) for the results of the analysis comprise digital numbers and/or letters.

3. Portable device (1) in accordance with claim 1 or 2 in which the display means (4) for the results of the analysis comprise at least one light indicator.

4. Portable device (1) in accordance with any of the above claims in which the display means (4) comprise a first, a second and a third light indicator.

5. Portable device (1) in accordance with any of the above claims, wherein the lid (T) is
adapted to be affixed to the specimen collection cup by screwing it on to the specimen collection cup.

6. Portable Device (1) in accordance with any of the above claims , which comprises means for measuring the temperature in the pH electrode (2a) and the reference electrode (2b).

7. Use of a portable device in accordance with any of claims 1-6, for the measurement of pH in a sample wherein the sample is a fluid of biological origin.

8. Use of a portable device in accordance with claim 7, for the measurement of pH in a urine sample.

9. Use of a portable device in accordance with any of claims 1-6, for the measurement of pH in a water sample.

10. Method for measuring the pH of a sample with the portable device (1) according to claim 1, the method comprising the following steps:
measuring the pH by electrochemical measurement of the sample with the portable device of claim 1, and
providing an indication of the results of the measurement depending on what is found within at least one range of values.

## Patentansprüche

1. Tragbare Vorrichtung (1) zur Analyse des pH-Werts einer Probe durch elektrochemische Messung, welche folgendes umfasst:
• einen Probensammelbecher (5), der konfiguriert ist, um die Probe zu enthalten,
• einen Deckel (T), der konfiguriert ist, um an dem Probensammelbecher (5) angebracht zu werden, und
• einen elektrochemischen pH-Sensor (2),
wobei die tragbare Vorrichtung (1) **dadurch gekennzeichnet ist, dass** der Deckel (T) folgendes umfasst:
∘ einen Mikroprozessor (3), der mit dem elektrochemischen pH-Sensor (2) verbunden ist und konfiguriert ist, um den pH-Wert der Probe beruhend auf einer Messung durch den elektrochemischen pH-Sensor (2) zu analysieren,
∘ ein Anzeigemittel (4), das mit dem Mikroprozessor (3) verbunden ist, um die Ergebnisse der Analyse anzuzeigen,
∘ eine Stromquelle (F) und/oder eine Batterie (B), die mit dem Mikroprozessor (3) und dem Anzeigemittel (4) verbunden ist bzw. sind, und
wobei der elektrochemische pH-Sensor (2) von dem Deckel (T) herausragt, um in Kontakt mit der in dem Probensammelbecher (5) enthaltenen Probe zu kommen, wobei der elektrochemische pH-Sensor (2) folgendes umfasst:
∘ eine pH-Elektrode (2a), und
∘ eine Referenzelektrode (2b).

2. Tragbare Vorrichtung (1) nach Anspruch 1, bei der das Anzeigemittel (4) für die Ergebnisse der Analyse digitale Zahlen und/oder Buchstaben umfasst.

3. Tragbare Vorrichtung (1) nach Anspruch 1 oder 2, bei der das Anzeigemittel (4) für die Ergebnisse der Analyse mindestens eine Lichtanzeige umfasst.

4. Tragbare Vorrichtung (1) nach einem der obigen Ansprüche, in dem das Anzeigemittel (4) einen ersten, einen zweiten und einen dritten Lichtanzeige umfasst.

5. Tragbare Vorrichtung (1) nach einem der obigen Ansprüche, wobei der Deckel (T) angepasst ist, um an dem Probensammelbecher angebracht zu werden, indem er an den Probensammelbecher angeschraubt wird.

6. Tragbare Vorrichtung (1) nach einem der obigen Ansprüche, welche ein Mittel zum Messen der Temperatur in der pH-Elektrode (2a) und der Referenzelektrode (2b) umfasst.

7. Verwendung einer tragbaren Vorrichtung nach einem der Ansprüche 1 bis 6, zur Messung des pH-Werts in einer Probe, wobei die Probe eine Flüssigkeit biologischen Ursprungs ist.

8. Verwendung einer tragbaren Vorrichtung nach Anspruch 7, zur Messung des pH-Werts in einer Urinprobe.

9. Verwendung einer tragbaren Vorrichtung nach einem der Ansprüche 1 bis 6, zur Messung des pH-Werts in einer Wasserprobe.

10. Verfahren zur Messung des pH-Werts einer Probe mit der tragbaren Vorrichtung (1) nach Anspruch 1, wobei das Verfahren folgende Schritte umfasst:
Messen des pH-Werts durch elektrochemische Messung der Probe mit der tragbaren Vorrichtung des Anspruchs 1, und
Bereitstellen einer Angabe der Messergebnisse in Abhängigkeit davon, was sich in mindestens einem Wertebereich befindet.

## Revendications

1. Dispositif portable (1) pour analyser le pH d'un échantillon par mesure électrochimique, qui comprend :
• une coupelle de prélèvement d'échantillons (5) configurée pour contenir l'échantillon,
• un couvercle (T) configuré pour être attaché à la coupelle de prélèvement d'échantillons (5), et
• un capteur électrochimique de pH (2),
dans lequel le dispositif portable (1) est **caractérisé en ce que** le couvercle (T) comprend
∘ un microprocesseur (3) relié au capteur électrochimique de pH (2) et configuré pour analyser le pH de l'échantillon sur la base d'une mesure effectuée par le capteur électrochimique de pH (2),
∘ un moyen d'affichage (4) relié au microprocesseur (3) pour afficher les résultats de l'analyse,
∘ une source d'alimentation (F) et / ou une batterie (B) reliée(s) au microprocesseur (3) et au moyen d'affichage (4), et
dans lequel le capteur électrochimique de pH (2) fait saillie par rapport au couvercle (T) de manière à venir en contact avec l'échantillon contenu dans la coupelle de prélèvement d'échantillons (5), le capteur électrochimique de pH (2) comprenant :
∘ une électrode de pH (2a), et
∘ une électrode de référence (2b).

2. Dispositif portable (1) selon la revendication 1, dans lequel le moyen d'affichage (4) pour les résultats de l'analyse comprend des chiffres digitaux et/ou des lettres.

3. Dispositif portable (1) selon la revendication 1 ou 2, dans lequel le moyen d'affichage (4) pour les résultats de l'analyse comprend au moins un indicateur lumineux.

4. Dispositif portable (1) selon l'une quelconque des revendications ci-dessus dans lequel le moyen d'affichage (4) comprend un premier, un deuxième et un troisième indicateur lumineux.

5. Dispositif portable (1) selon l'une quelconque des revendications ci-dessus, dans lequel le couvercle (T) est adapté pour être fixé à la coupelle de prélèvement d'échantillons en le vissant sur la coupelle de prélèvement d'échantillons.

6. Dispositif portable (1) selon l'une quelconque des revendications ci-dessus, qui comprend un moyen pour mesurer la température dans l'électrode de pH (2a) et l'électrode de référence (2b).

7. Utilisation d'un dispositif portable selon l'une quelconque des revendications 1 à 6, pour la mesure du pH dans un échantillon où l'échantillon est un fluide d'origine biologique.

8. Utilisation d'un dispositif portable selon la revendication 7, pour la mesure du pH dans un échantillon d'urine.

9. Utilisation d'un dispositif portable selon l'une quelconque des revendications 1 à 6, pour la mesure du pH dans un échantillon d'eau.

10. Procédé de mesure du pH d'un échantillon avec le dispositif portable (1) selon la revendication 1, le procédé comprenant les étapes suivantes :
mesurer le pH par mesure électrochimique de l'échantillon avec le dispositif portable de la revendication 1, et
fournir une indication des résultats de la mesure en fonction de ce qui se trouve dans au moins une plage de valeurs.
